Europäisches Patentamt

European Patent Office

Office européen des brevets

(11) Veröffentlichungsnummer: **0 087 815**
**B1**

(12) **EUROPÄISCHE PATENTSCHRIFT**

(45) Veröffentlichungstag der Patentschrift:
**14.05.86**

(21) Anmeldenummer: **83102028.4**

(22) Anmeldetag: **02.03.83**

(51) Int. Cl.⁴: **A 61 F 13/12,** A 61 F 7/10,
A 61 H 33/06, A 61 H 35/00

(54) Haube zum Anpressen einer zur therapeutischen Behandlung der Kopfhaut mit Kälte vorgesehenen Kompresse.

(30) Priorität: **03.03.82 DE 8205878 U**

(43) Veröffentlichungstag der Anmeldung:
**07.09.83 Patentblatt 83/36**

(45) Bekanntmachung des Hinweises auf die Patenterteilung:
**14.05.86 Patentblatt 86/20**

(84) Benannte Vertragsstaaten:
**AT BE CH DE FR GB IT LI LU NL SE**

(56) Entgegenhaltungen:
**DE - B - 1 541 303**
**DE - C - 254 250**
**DE - C - 660 433**
**GB - A - 446 788**
**US - A - 3 709 225**

(73) Patentinhaber: **Hospipharm Produktions- und Vertriebsges. mbH, Gerresheimer-Landstrasse 110, D-4000 Düsseldorf 12 (DE)**

(72) Erfinder: **Hecht, Manfred, Gerresheimer-Land-Strasse 110, D-4000 Düsseldorf 12 (DE)**

(74) Vertreter: **Ruschke, Hans Edvard et al, Patentanwälte Dipl.-Ing. Olaf Ruschke Dipl.-Ing. Hans E. Ruschke Dipl.-Ing. Jürgen Rost Dipl.-Chem. Dr. U. Rotter Pienzenauerstrasse 2, D-8000 München 80 (DE)**

## Beschreibung

Die vorliegende Neuerung betrifft eine Haube zum Anpressen einer zur therapeutischen Behandlung der Kopfhaut mit Kälte vorgesehenen Kompresse nach dem Oberbegriff des Patentanspruches 1. Eine derartige Haube ist in der DE-C-254250 beschrieben. Sie zeigt einen Halter für Kopfeisbeutel, der mützenförmig ausgebildet ist und mit der Hilfe von Bändern am Kopf eines Patienten befestigt wird. Dabei ist der mützenförmige Halter so ausgebildet, dass zwischen zwei deckelförmigen Teilen, die in üblicher Weise mit dem Rand der Mütze vernäht sind, ein Eisbeutel angeordnet werden kann. Zu diesem Zweck weist das obere deckelförmige Teil einen länglichen Einschnitt auf, durch den der Eisbeutel eingeführt werden kann.

Bei bestimmten Krebsformen hat eine medikamentöse Chemotherapie gute Erfolge gezeigt. Die hierbei verabreichten Medikamente haben jedoch die unangenehme Nebenwirkung, dass sie zum totalen Haarausfall führen können, weil sie die Haarwurzelzellen schädigen. Um diesen unangenehmen Nebeneffekt möglichst gering zu halten, hat man sich mit gutem Erfolg darum bemüht, insbesondere während der Phasen höchster Konzentration der Medikamente im Blut des Patienten die Blutzufuhr zu den Haarwurzeln weitgehend zu reduzieren, um dadurch eine Schädigung der Haarwurzel so gering wie möglich zu halten. Die Blutzufuhr zu den Haarwurzeln kann dadurch reduziert werden, dass die Kopfhaut während der Chemotherapie unterkühlt wird. Dies führt dazu, dass infolge der bewirkten Gefässverengung die Menge des anflutenden Medikamentes reduziert wird. Gleichzeitig wird die Zellteilung verlangsamt und somit die Einbaurate des Medikamentes in die Zellkerne vermindert. Beispielsweise kann der Haarausfall bei abkühlender Kopfhaut auf unter +20°C in Abhängigkeit von der jeweiligen Behandlung völlig vermieden oder zumindest stark reduziert werden.

Aus dem DE-GU-M 8115207 ist eine Kompresse zur Unterkühlung der Kopfhaut bekannt. Im wesentlichen besteht diese Kompresse aus mehreren von einer Kunststoffolie umgrenzten, segmentartigen flachen Kammern, die mit einer gelartigen Masse gefüllt sind. Wenn die Kammern mit der gelartigen Masse mit der Kopfhaut in thermischen Kontakt gelangen, wird der Kopfhaut Wärme entzogen. Beispielsweise lässt sich mit derartigen Kompressen die Temperatur der Kopfhaut über einen Zeitraum von etwa 40 Minuten absenken.

Es ist ganz allgemein bekannt, die Temperatur der Kopfhaut dadurch weiter abzusenken, dass die Kompresse durch eine elastische Bandage an die Kopfhaut des Patienten angepresst wird. Dabei besteht ein Nachteil darin, dass das Anlegen einer derartigen Bandage sehr umständlich und zeitraubend ist.

Die Aufgabe der vorliegenden Erfindung besteht demgemäss darin, es zu ermöglichen, die Temperatur der Kopfhaut durch Anpressen der Kompresse in einer einfachen und weniger zeitraubenden Weise weiter und länger abzusenken.

Diese Aufgabe wird durch eine Haube zum Anpressen einer zur therapeutischen Behandlung der Kopfhaut mit Kälte vorgesehenen Kompresse gelöst, die durch die in dem kennzeichnenden Teil des Patentanspruches 1 angegebenen Merkmale gekennzeichnet ist.

Ein wesentlicher Vorteil der vorliegenden Neuerung besteht darin, dass durch die gleichmässige Anpressung der Kompresse durch die Haube eine sehr viel gleichmässigere Temperaturverteilung auf der Kopfhaut erreichbar ist.

Vorteilhafterweise kann die Kopfhaut bei der Verwendung der neuerungsgemässen Haube auf Temperaturen abgekühlt werden, die noch tiefer liegen als die Temperaturen, die beim Anpressen der Komprese mit einer Bandage erreichbar sind, weil das Material der Haube wärmeisolierend ist.

Ein weiterer Vorteil der Neuerung besteht darin, dass infolge Elastizität der Haube extreme Druckstellen vermieden werden können.

Vorteilhafterweise ist die neuerungsgemässe Haube in einer äusserst einfachen und schnellen Weise durch das Schliessen von Klettverschlüssen am Kopf des Patienten befestigbar.

Beim Verschliessen der Klettverschlüsse bzw. beim Spannen der Haube wird ein Hochrutschen der Haube vorteilhafterweise durch einen Kinnriemen vermieden.

Vorteilhafterweise ist die neuerungsgemässe Haube in einfacher Weise an beliebige Kopfgrössen und Kopfformen anpassbar.

Im folgenden wird die Neuerung im Zusammenhang mit den Figuren näher beschrieben. Es zeigt:

Fig. 1 eine Vorderansicht der Haube, wobei die einzelnen Klettverschlüsse geöffnet sind, und

Fig. 2 die Rückansicht der Haube, wobei die einzelnen Klettverschlüsse ebenfalls geöffnet sind.

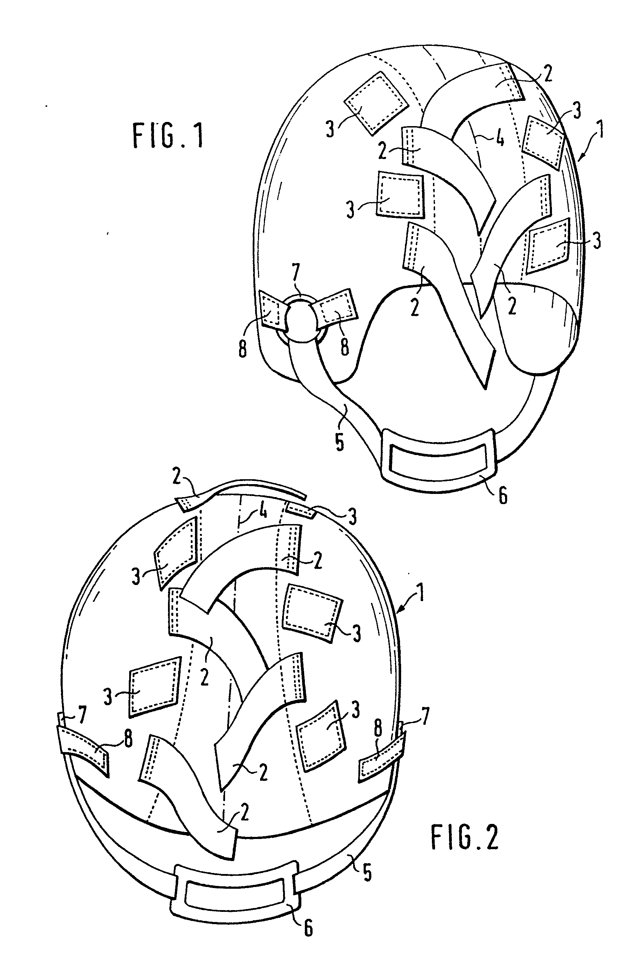

Die in der Fig. 1 dargestellte Haube 1 besteht aus einem hochelastischen Material, bei dem es sich beispielsweise um ein wärmeisolierendes Schaumstoffmaterial, vorzugsweise um Neopren, handelt. Die Haube 1 wird auf den mit einer Kompresse versehenen Kopf des Patienten aufgesetzt. An der Haube 1 sind in der aus der Fig. 1 ersichtlichen Weise eine Reihe von Klettverschlüssen vorgesehen. Jeweils ein Klettverschluss besteht aus einem an der Haube befestigten Streifen 2 und einem an der Haube befestigten Bereich 3.

Vorzugsweise sind die Enden der Streifen 2 und die Bereiche 3 an der Haube 1 vernäht, wie dies in den Fig. 1 und 2 durch gepunktete Linien angedeutet ist. Dabei sind die Befestigungsstelle des Streifens 2 und der Bereich 3 voneinander beabstandet, so dass beim Schliessvorgang des Klettverschlusses, bei dem der Streifen 2 in Richtung auf den Bereich 3 gezogen und an diesem befestigt wird, das elastische Material der Haube 1 gespannt wird. Vorzugsweise sind die Klettverschlussteile 2, 3 symmetrisch zu einer gedachten Linie 4 angeordnet und von dieser beabstandet, die die Haube 1 in zwei gleiche Teile teilt. Um eine möglichst gleichmässige Anpressung der Kompresse zu erzielen, sind die Klettverschlussteile 2, 3 vorteilhafterweise so angeordnet, dass neben jeweils einem Bereich 3 eines Klettverschlusses die Befestigungsstelle des Streifens 2 des nächstfolgenden Klettverschlusses angeordnet ist.

Aus der Fig. 2, die die Rückseite der Haube 1 zeigt, ist ersichtlich, dass auch an der Rückseite die jeweiligen Klettverschlussteile 2, 3 symmetrisch zur Linie 4 angeordnet sind. Vorzugsweise weisen die einzelnen Klettverschlussteile entlang einer parallel zur Linie 4 verlaufenden Linie jeweils die gleichen Abstände voneinander auf.

Es ist auch denkbar, an der Stelle der Klettverschlüsse 2, 3 andere Verschlüsse vorzusehen, mit denen das elastische Material der Haube gespannt werden kann. Beispielsweise eignen sich als derartige Verschlüsse Riemen oder Bänder.

Um beim Spannen der Haube 1 bzw. beim Schliessen der Verschlüsse 2, 3 ein Hochrutschen der Haube 1 zu vermeiden, ist vorzugsweise ein Kinnriemen 5 vorgesehen. Die Enden dieses vorzugsweise bezüglich seiner Länge verstellbaren Kinnriemens 5 sind an sich gegenüberliegenden unteren Bereichen der Haube 1 befestigt. Beispielsweise sind die Enden des Kinnriemens 5 an Bereichen der Haube befestigt, die unterhalb der Ohren des die Haube tragenden Patienten liegen.

Um eine Verschwenkbarkeit des Kinnriemens 5 zu ermöglichen, sind die Enden des Kinnriemens 5 vorzugsweise in der aus der Fig. 1 ersichtlichen Weise gleitbar an einem Ring 7 angeordnet, der an den zuvor erwähnten Bereichen der Haube 1 befestigt ist. Beispielsweise ist der vorzugsweise aus Metall bestehende Ring 7 durch die Textilstreifen 8 an der Haube 1 vernäht, wie dies in der Fig. 1 durch gepunktete Linien angedeutet ist.

Um ein Verrutschen des Kinnriemens 5 zu vermeiden, ist an ihm ein vorzugsweise aus Kunststoff bestehendes Formteil 6 angeordnet, in das das Kinn des die Haube tragenden Patienten eingreift. Dabei ist das Formteil 6 auf dem Kinnriemen 5 vorzugsweise in Richtung der Länge des Riemens verschiebbar.

Im folgenden wird die Art und Weise beschrieben, in der die neuerungsgemässe Haube 1 befestigt wird. Zunächst wird auf die Kopfhaut des Patienten eine die Unterkühlung bewirkende Kompresse aufgebracht. Auf diese Kompresse wird die Haube 1 gestülpt. Anschliessend wird der Kinnriemen 5 derart befestigt, dass das Kinn des Patienten in das Formteil 6 eingreift. Schliesslich wird die Haube 1 durch Verschliessen der Klettverschlüsse 2, 3 derart gespannt, dass die Kompresse gleichmässiger und besser an die Kopfhaut des Patienten angepresst wird.

Mit der Hilfe der neuerungsgemässen Haube kann die Temperatur der Kopfhaut eines Patienten etwa 112 Minuten lang auf Werte abgesenkt werden, die in einem Bereich von etwa 15 bis 20°C liegen.

Vorzugsweise ist das Nackenteil der neuerungsgemässen Haube besonders weit nach unten gezogen, um in diesem Bereich eine besonders gute Anpressung der Kompresse zu bewirken. Eine Anpressung der Kompresse in diesem Bereich ist besonders wichtig, um hier eine Verengung der Hauptversorgungsgefässe zu bewirken.

**Patentansprüche**

1. Haube zum Anpressen einer zur therapeutischen Behandlung der Kopfhaut mit Kälte vorgesehenen Kompresse, wobei die Haube über die Kompresse stülpbar ist, dadurch gekennzeichnet, dass die Haube (1) aus einem elastischen Material besteht und dass auf der Aussenfläche der Haube (1) Verschlüsse (2, 3) vorgesehen sind, mit denen das elastische Material der Haube (1) dadurch spannbar ist, dass einzelne Teilbereiche der Haube (1) aufeinander zu bewegbar sind.

2. Haube nach Anspruch 1, dadurch gekennzeichnet, dass als elastisches Material ein isolierendes Schaumstoffmaterial vorgesehen ist.

3. Haube nach Anspruch 1 oder 2, dadurch gekennzeichnet, dass als Material Neopren vorgesehen ist.

4. Haube nach einem der Ansprüche 1 bis 3, dadurch gekennzeichnet, dass als Verschlüsse Klettverschlüsse vorgesehen sind, die jeweils aus einem an einem Teilbereich der Haube (1) befestigten Streifen (2) und einem an einem anderen, von dem einen Teilbereich beabstandeten Teilbereich der Haube (1) befestigten Bereich (3) bestehen.

5. Haube nach Anspruch 4, dadurch gekennzeichnet, dass die Befestigungsstelle eines Streifens (2) und ein Bereich (3) jeweils eines Klettverschlusses symmetrisch zu der die Haube (1) in etwa zwei gleiche Teile teilenden gedachten Linie (4) angeordnet sind.

6. Haube nach Anspruch 4 oder 5, dadurch gekennzeichnet, dass die Streifen (2) bzw. die Bereiche (3) mehrerer Klettverschlüsse etwa entlang von weiteren Linien angeordnet sind, die etwa parallel zur Linie (4) verlaufen und von dieser durch etwa gleiche Abstände entfernt sind.

7. Haube nach Anspruch 6, dadurch gekennzeichnet, dass entlang der weiteren Linie jeweils neben einem Bereich (3) eines Klettverschlusses bzw. neben einer Befestigungsstelle eines Streifens (2) eines Klettverschlusses eine Befestigungsstelle eines Streifens (2) eines benachbarten Klettverschlusses bzw. ein Bereich (3) eines benachbarten Klettverschlusses angeordnet ist.

8. Haube nach Anspruch 6 oder 7, dadurch gekennzeichnet, dass die Bereiche (3) bzw. die Befestigungsstellen der Streifen (2) der Klettverschlüsse entlang der weiteren Linien etwa die gleichen Abstände voneinander aufweisen.

9. Haube nach einem der Ansprüche 1 bis 3, dadurch gekennzeichnet, dass als Verschlüsse an einzelnen Teilbereichen der Haube (1) befestigte Riemen oder Bänder vorgesehen sind.

10. Haube nach einem der Ansprüche 1 bis 9, dadurch gekennzeichnet, dass zum Fixieren der Haube (1) an dem mit einer Kompresse versehenen Kopf eines Patienten ein Kinnriemen (5) vorgesehen ist.

11. Haube nach Anspruch 10, dadurch gekennzeichnet, dass die Enden des Kinnriemens (5) an sich gegenüberliegenden Stellen der Haube (1) befestigt sind, die sich etwa in der Nähe des unteren Endes der Haube befinden.

12. Haube nach Anspruch 10 oder 11, dadurch gekennzeichnet, dass der Kinnriemen (5) verstellbar ist.

13. Haube nach einem der Ansprüche 10 bis 12, dadurch gekennzeichnet, dass jeweils ein Ende des Kinnriemens (5) an einem Ring (7) gleitbar angeordnet ist und dass die Ringe (7) in der Nähe der unteren Ränder der Haube (1) befestigt sind.

14. Haube nach Anspruch 13, dadurch gekennzeichnet, dass die Ringe (7) durch an der Haube (1) vernähte Textilstreifen (8) befestigt sind.

15. Haube nach einem der Ansprüche 10 bis 14, dadurch gekennzeichnet, dass an dem Kinnriemen (5) ein Formteil (6) vorgesehen ist, das eine Vertiefung zur Aufnahme des Kinns des Patienten aufweist.

16. Haube nach Anspruch 15, dadurch gekennzeichnet, dass das Formteil (6) an dem Kinnriemen (5) in Längsrichtung des Kinnriemens (5) verschiebbar ist.

17. Haube nach Anspruch 15 oder 16, dadurch gekennzeichnet, dass das Formteil (6) aus Kunststoff besteht.

## Claims

1. Hood for pressing in position a compress for the therapeutic cold treatment of the scalp, the hood being adapted to be placed over said compress, characterized by hood (1) consisting of an elastic material and having on the outer surface thereof closures (2, 3) suited to tension the elastic material of hood (1) by moving portions of hood (1) towards each other.

2. Hood as in claim 1, characterized by the elastic material being an insulative foam material.

3. Hood as in claim 1 or 2, characterized by the material being neoprene.

4. Hood as in any one of claims 1 to 3, characterized by said closures being thistle-cloth closures each comprising a strap secured to portion of hood (1) and an area (3) secured to another portion of hood (1), said another portion being spaced from said first mentioned portion.

5. Hood as in claim 4, characterized by the points of securement of strap (2) and of an area (3) of any one thistle-cloth closure being symmetrical relative to an imaginary line (4) dividing hood (1) into two substantially equal parts.

6. Hood as in claim 4 or 5, characterized by straps (2) or areas (3) of plurality of thistle-cloth closures being disposed substantially along additional lines substantially parallel to and substantially equidistant from line (4).

7. Hood as in claim 6, characterized by each area (3) of a thistle-cloth closure or each place of securement of a strap (2) of a thistle-cloth closure having disposed next thereto along said additional line a place of securement of a strap (2) of an adjacent thistle-cloth closure or an area (3) of an adjacent thistle-cloth closure, respectively.

8. Hood as in claim 6 or 7, characterized by areas (3) or the places of securement of straps (2) of said thistle-cloth closures being substantially equidistant along said additional lines.

9. Hood as in any one of claims 1 to 3, characterized by individual portions of hood (1) having straps or bands secured thereto as closures.

10. Hood as in any one of claims 1 to 9, characterized by a chin strap (5) being provided to hold hood (1) in position on a patient's head and on a compress placed on the head.

11. Hood as in claim 10, characterized by the ends of a chin strap (5) being secured on hood (1) in positions located opposite each other and substantially adjacent the lower end of the hood.

12. Hood as in claim 10 or 11, characterized by chin strap (5) being adjustable.

13. Hood as in any one of claims 10 to 12, characterized by one end of each chin strap (5) being movably secured to a ring (7) and by rings (7) being secured adjacent the bottom edges of hood (1).

14. Hood as in claim 13, characterized by rings (7) being secured by textile straps (8) sewn on hood (1).

15. Hood as in any one of claims 10 to 14, characterized by a molded element (6) on chin strap (5) and having therein an opening to receive the patient's chin.

16. Hood as in claim 15, characterized by molded element (6) being movable on chin strap (5) in the longitudinal direction thereof.

17. Hood as in claim 15 or 16, characterized by molded element (6) being made of a plastics material.

## Revendications

1. Bonnet pour l'application d'une compresse prévue pour le traitement thérapeutique par le froid du cuir chevelu, le bonnet pouvant être passé au-dessus de la compresse, caractérisé en ce que le bonnet (1) est constitué d'une matière élastique et en ce que sur la face externe du bonnet (1) sont prévues des fermetures (2, 3) par lesquelles la matière élastique du bonnet (1) peut être tendue par le fait que différentes zones partielles du bonnet (1) sont déplaçables l'une sur l'autre.

2. Bonnet suivant la revendication 1, caractérisé en ce qu'on prévoit une matière mousse isolante à titre de matière élastique.

3. Bonnet suivant la revendication 1 ou 2, caractérisé en ce que du néoprène est prévu comme matière.

4. Bonnet suivant une des revendications 1 à 3, caractérisé en ce que, comme fermetures, on prévoit des fermetures à crampons, qui sont chacune constituées d'une bande (2), fixée sur une zone partielle du bonnet (1), et d'une zone (3) fixée sur une autre zone partielle du bonnet (1) située à distance de la première zone partielle.

5. Bonnet suivant la revendication 4, caractérisé en ce que l'emplacement de fixation d'une bande (2) et une zone (3) d'une fermeture à crampons respective sont agencés de manière symétrique par rapport à ligne imaginaire (4) qui divise le bonnet (1) en deux parties approximativement égales.

6. Bonnet suivant la revendication 4 ou 5, caractérisé en ce que les bandes (2) et respectivement les zones (3) de plusieurs fermetures à crampons sont agencées approximativement le long d'autres lignes qui sont disposées environ parallèlement à la ligne (4) et sont éloignées de cette dernière de distances approximativement égales.

7. Bonnet suivant la revendication 6, caractérisé en ce que, le long des autres lignes, est agencé chaque fois, à côté d'une zone (3) d'une fermeture à crampons ou respectivement d'un emplacement de fixation d'une bande (2) d'une fermeture à crampons, un emplacement de fixation d'une bande (2) d'une fermeture à crampons voisine ou respectivement une zone (3) d'une fermeture à crampons voisine.

8. Bonnet la revendication 6 ou 7, caractérisé en ce que les zones (3) et respectivement les emplacements de fixation des bandes (2) des fermetures à crampons présentent, le long des autres lignes, approximativement les mêmes distances mutuelles.

9. Bonnet suivant une des revendications 1 à 3, caractérisé en ce que, comme fermetures, on prévoit des courroies ou sangles fixées sur différentes zones partielles du bonnet (1).

10. Bonnet suivant une des revendications 1 à 9, caractérisé en ce qu'une mentonnière (5) est prévue pour la fixation du bonnet (1) sur la tête, garnie d'une compresse, d'un patient.

11. Bonnet suivant la revendication 10, caractérisé en ce que les extrémités de la mentonnière (5) sont fixées en des endroits opposés du bonnet (1) qui se trouvent environ à proximité de l'extrémité inférieure du bonnet.

12. Bonnet suivant la revendication 10 ou 11, caractérisé en ce que la mentonnière (5) est ajustable.

13. Bonnet suivant une des revendications 10 à 12, caractérisé en ce que chaque extrémité de la mentonnière (5) est agencée de manière à pouvoir glisser sur un anneau (7) et en ce que les anneaux (7) sont fixés à proximité des bords inférieurs du bonnet (1).

14. Bonnet suivant la revendication 13, caractérisé en ce que les anneaux (7) sont fixés par des bandes en matière textile (8) cousues sur le bonnet (1).

15. Bonnet suivant une des revendications 10 à 14, caractérisé en ce que sur la mentonnière (5) est prévue une pièce façonnée (6) qui présente un évidement pour recevoir le menton du patient.

16. Bonnet suivant la revendication 15, caractérisé en ce que la pièce façonnée (6) est capable de coulisser sur la mentonnière (5) suivant la direction longitudinale de la mentonnière (5).

17. Bonnet suivant la revendication 15 ou 16, caractérisé en ce que la pièce façonnée (6) est constituée en une substance synthétique.

FIG.1

FIG.2